# EUROPÄISCHE PATENTANMELDUNG

(11) **EP 1 645 296 A1**
(43) Veröffentlichungstag der Anmeldung: **12.04.2006**
(21) Anmeldenummer: 05021110.1
(22) Anmeldetag: 27.09.2005
(51) Int. Cl.: A61L 12/08, A61L 12/14, C11D 3/00, B29D 11/00

(54) **Aufbewahrungs-, Reinigungs- und/oder Pflegelösung für Kontaktlinsen, Farbstoff und Verfahren zum Einfärben von Kontaktlinsen**

(30) Priorität: 29.09.2004 DE 102004047156
(71) Anmelder: Kloth, Stefan, 24226 Heikendorf (DE)
(72) Erfinder: Kloth, Stefan, 24226 Heikendorf (DE)
(74) Vertreter: Hauck, Graalfs, Wehnert, Döring, Siemons

(57) **Zusammenfassung**

Pflege-, Reinigungs- und/oder Aufbewahrungslösung für Kontaktlinsen, die einen in der Pflege- und/oder Aufbewahrungslösung löslichen, für Auge und Körper verträglichen, abspülbaren Farbstoff zum Einfärben der Kontaktlinsen enthält.

## Beschreibung

Die Erfindung bezieht sich auf eine Pflege- und/oder Reinigungs- und/oder Aufbewahrungslösung für Kontaktlinsen nach dem Patentanspruch 1 sowie auf einen Farbstoff für eine Pflege- und/oder Reinigungs- und/oder Aufbewahrungslösung nach Anspruch 2 sowie auf ein Verfahren zum Färben von Hydrogel-Kontatklinsen nach Anspruch 8.

Die am meisten verbreiteten Kontaktlinsen sind sogenannte weiche oder Hydrogel-Linsen. Sie bestehen aus Polymeren und haben eine hohen Wassergehalt z.B. zwischen 38 und 74 Prozent. Sie werden üblicherweise im Gießform verfahren (seltener im Schleudergussverfahren) hergestellt, bei dem ein flüssiges Monomer in eine Form eingebracht und durch Wärme oder UV-Strahlung ausgehärtet wird. Es entsteht ein verfestigtes Polymer, welches anschließend hydratisiert und sterilisiert wird. Erst durch Hydratisierung wird die Kontaktlinse weich.

Die Kontaktlinsen werden zumeist schwach eingefärbt, damit der Benutzer die Linse besser sehen und anfassen kann. Diese leichte Färbung wird auch als "Handling Tint" bezeichnet. Bei eingesetzter Kontaktlinse ist diese Färbung kosmetisch nicht wirksam.

Es sind auch farbige bzw. gefärbte Kontaktlinsen bekannt. Sie werden aus kosmetischen Gründen verwendet. Sie werden unterschieden in farbverstärkende und farbverändernde Kontaktlinsen. Die farbverstärkenden Linsen sind leichter gefärbt als die farbverändernden. Die leichter gefärbten Linsen sollen die vorhandene Augenfarbe lediglich verstärken. Farbverändernde Linsen sind meist kräftig gefärbt und zum Beispiel mit einer Farbe bedruckt. In diesem Fall muss der Pupillenbereich frei von Farbe sein, da sonst das Sehvermögen ganz oder teilweise eingeschränkt ist.

Aus einer größeren Reihe von Druckschriften zum Stand der Technik ist bekannt, Hydrogel-Kontaktlinsen dadurch zu färben, dass in einem von mehreren Verfahrensschritten diese in eine Flüssigkeit eingetaucht werden, welche eine reaktive Farbe enthält. Die Farbe verbindet sich mit dem Polymer während der Polymerisation. Ein solches Verfahren ist etwa in US 5,938,795, US 5,151,106, US 5,292,350 oder US 4,891,046 beschrieben.

Im Zusammenhang mit dem Färben von Kontaktlinsen ist auch bekannt geworden, der Farblösung ein Mittel zuzugeben, um das Polymer der Linse aufzuquellen bzw. entsprechende Poren zu öffnen (US 4,494,954 oder WO 0045198).

Aus US 4,733,959 oder US 4,518,390 sind Vorrichtungen bekannt geworden (Masken), um Kontaktlinsen partiell einzufärben. Aus US 4,865,439 oder US 4,903,052 ist bekannt geworden Kontaktlinsen im Irisbereich mit Hilfe eines Porenöffners opak einzufärben, wobei der Pupillenbereich frei bleibt.

Aus US 5,021,068 ist bekannt geworden, einer wässrigen Farblösung zum Färben von Hydrogel-Kontaktlinsen einen Katalysator in Form eines quaternären Phosphoniumsalzes hinzuzufügen. Der Farbstoff ist reaktiv und bindet sich an das Monomer des polymeren Materials. Zur Durchführung des Verfahrens werden die Kontaktlinsen zunächst mit entionisiertem Wasser gespült und in eine trockene Schale gegeben. In diese wird eine basische Lösung sowie die Farblösung eingegeben. Die Schale wird anschließend geschüttelt. Danach werden die Linsen mit entionisiertem Wasser gespült und in einer zehnprozentigen wässerigen Glycerinlösung für zwei Stunden extrahiert. Danach werden die Linsen noch einmal gespült.

Aus US 5,451,237 ist ein Verfahren bekannt geworden, mit dem die Bildung von Eiweißablagerungen auf der Oberfläche oder im Inneren der Matrix einer Kontaktlinse verhindert werden soll. Das hierfür verwendete Mittel ist farbig und dringt in die Linse ein.

Die beschriebenen bekannten Verfahren sind Teil des industriellen Herstellungsprozesses, das heißt die Einfärbung der Hydrogel-Kontaktlinsen erfolgt während ihrer Herstellung, entweder durch Einfärbung des Materials vor der Formgebung, während der Formgebung oder auch danach. Die Farben beschränken sich naturgemäß auf Standardfarben und lassen sich nicht individuell auf den jeweiligen Nutzer einstellen.

Der Erfindung liegt die Aufgabe zugrunde, eine Möglichkeit anzugeben, mit welcher eine sehr einfache, kostengünstige und im wesentlichen individuelle Einfärbung von Kontaktlinsen zu kosmetischen Zwecken (oder aber auch zu "Spaßzwecken" (s.u.) oder als "Sonnenbrille") vorgenommen werden kann. Es gibt heute Wegwerflinsen, die preislich sehr günstig sind. Deshalb ist es für den Nutzer kein Problem, wenn er ein Paar Linsen zweckentfremdet und z.B. dunkelbraun einfärbt. Dann hat er z.B. für das Skifahren in gleißendem Licht ein Paar "Sonnenbrillen-Linsen". Nach dem Skifahren wirft er die Linsen weg, um abends wieder "klare" oder leicht gefärbte Linsen zu tragen.

Diese Aufgabe wird durch die Merkmale der Patentansprüche 1, 2 und 8 gelöst.

Kontaktlinsen werden, wenn sie aus dem Auge entfernt werden bis zu ihrem erneuten Einsatz zumeist in einer Pflege-, Reinigungs- und/oder Aufbewahrungslösung gehalten. Die heutzutage verwendeten Pflege-, Reinigungs- und/oder Aufbewahrungslösungen sind sogenannte isotonische Lösungen, die den gleichen osmotischen Druck wie die hydratisierten weichen Kontaktlinsen bzw. das menschliche Auge aufweisen. Nach Möglichkeit soll es beim Tragen der Kontaktlinsen nicht zu einer osmotischen Wirkung Kochsalzgehalt auf und sind naturgemäß entionisiert und zumeist mit einer antibakteriell wirkenden Substanz versehen. Reine Aufbewahrungslösungen beinhalten meist keine zusätzlich antibakteriell wirksamen Substanzen, sondern nur 0,9 % NaCl. Derartige Pflege-, Reinigungs- und/oder Aufbewahrungslösungen sind im Handel erhältlich. Es ist jedoch auch denkbar, herkömmliches Leitungswasser zu verwenden, das vom Benutzer mit entsprechenden Zusätzen als Pflege- und Aufbewahrungsmittel geeignet gemacht wird.

Bei einem anderen bekannten Verfahren zur Pflege und Aufbewahrung von Kontaktlinsen wird in zwei Stufen vorgegangen. In der ersten Stufe werden die Linsen in z.B. dreiprozentigem Wasserstoffperoxid gehalten und in einer zweiten Stufe mit Hilfe von einem Neutralisator (z.B. Katalase, Platin etc.) neutralisiert. Das Wasserstoffperoxid wird unter Abgabe von Sauerstoff in Wasser umgewandelt, wobei dem Wasser Kochsalz und andere Substanzen in entsprechender Menge zugesetzt ist, um isotonisch zu wirken.

Bei der Erfindung wurde erkannt, dass es möglich ist, Hydrogel-Kontaktlinsen auch vom Benutzer einfärben zu lassen, indem er eine Pflege-, Reinigungs- und/oder Aufbewahrungslösung verwendet, in der ein Farbstoff gelöst ist. Der Farbstoff, der chemisch nicht aktiv ist, muss für die Hornhaut und den menschlichen Organismus verträglich, abspülbar und geeignet sein, in einem vertretbaren Zeitraum, von zum Beispiel höchstens 8 Stunden, in die Matrix der Hydrogel-Linse einzudringen und diese zu färben. Die lösliche Farbe dringt mit zunehmender zeitlicher Lagerung in dem farbigen Pflegemittel immer tiefer in die Linse ein und erzeugt mit zunehmender Zeit eine intensivere Färbung. Der Benutzer kann auf diese Weise die Färbung der Linse selbst steuern, indem sie früher oder später aus der farbigen Lösung entnommen wird. Ebenso kann der Benutzer steuern, welcher Teil der Linse eingefärbt wird, indem er nur Teile der Linse dem farbigen Pflegemittels aussetzt (z.B. durch Eintunken nur der Hälfte der Linse in die farbige Lösung). Vor dem Einsetzen in das Auge ist die Linse nochmals mit klarer Lösung zu spülen, um überschüssige nicht eingedrungene Farbe zu entfernen, damit sie nicht unnötig in das Auge bzw. in den Organismus gelangt. Es versteht sich, dass die Farbe ungiftig und für das menschliche Auge, die Hornhaut und den Organismus verträglich und abspülbar sein muss. In Abhängigkeit von der Dicke der Linse kann - gerade bei kurzen Einfärbungszeiten - das Eindringen der Farbe ungleichmäßig vonstatten gehen, d.h. die Linse wird u.U. nicht 100% gleichmäßig eingefärbt. Dieser Effekt tritt bei längeren Einwirkzeiten nicht auf und ist zudem bei eingesetzter Linse nicht sichtbar.

Durch das Einlegen der Linsen in eine farbige Pflege- und Aufbewahrungslösung wird die Kontaktlinse mehr oder weniger stark eingefärbt. Wird die eingefärbte Linse über einen langen Zeitraum wieder ausschließlich in klarer Pflegelösung aufbewahrt, wird die Färbung etwas schwächer, bleibt jedoch nach wie vor erhalten. Bei manchen Farben verschwindet die Färbung vollständig. Durch regelmäßige Anwendung des farbigen Kontaktlinsen-Pflegemittels ist eine permanente Aufrechterhaltung des Einfärbungsgrades der Linsen möglich.

Die Einfärbung ist jedoch nicht so stark, dass sie das Sehvermögen des Nutzers beeinträchtigt. Ausnahme: Sonnenbrillen-Linse.

Durch serielle Anwendung von farbigen Pflege-, Reinigungs- und Aufbewahrungsmitteln unterschiedlicher Farben kann auch eine Mischfarbe ermöglicht werden, die so bei industriell gefertigten Linsen nicht vorhanden ist. Ferner können Pflegemittel verschiedener Farben bereits vor dem Eintunken der Linse zusammengemischt werden, sodass das Ergebnis, die Mischfarbe, dem Benutzer vor der Anwendung bekannt ist. Ferner kann, wie oben beschrieben, eine Linse nur partiell eingefärbt werden, sodass eine "Spaß-Linse" mit zwei mehr oder weniger voneinander getrennten unterschiedlichen Farbhälften vom Benutzer leicht herstellbar ist. Die Linsen für das rechte sowie für das linke Auge können mit unterschiedlichen Farben gefärbt werden. All dies kann industriell so nicht hergestellt werden.

Der Benutzer kann, wie nach Anspruch 1 ausgeführt, eine an sich fertige farbige Pflege-, Reinigungs- und/oder Aufbewahrungslösung verwenden, um seine Kontaktlinsen zu färben. Alternativ ist gemäß Anspruch 2 auch vorgesehen, zum Beispiel mit einer Pflegelösung einen oder mehrere Farbstoffe, die in einer Pflegelösung lösbar sind und die erwähnten Eigenschaften haben, dem Benutzer zur Verfügung zu stellen. Er kann sich dann die farbige Pflegelösung selbst mischen und vor allen Dingen den Färbungsgrad bestimmen oder durch Mischen mehrerer Farben eine neue Farbe erstellen. Es kann von Vorteil sein, wenn nach einer Ausgestaltung der Erfindung der Farbstoff seinerseits desinfizierend wirkt. Der Farbstoff kann nach einer weiteren Ausgestaltung der Erfindung in gelöster Form oder in pulverförmigen Zustand vorliegen. Es ist jedoch auch denkbar, den Farbstoff als Tablette vorzusehen bzw. ihn in einer in Flüssigkeit löslichen Tablette unterzubringen.

Ein Verfahren zum Färben von Hydrogel-Kontaktlinsen lässt sich auf einfache Weise erfindungsgemäß dadurch durchführen, dass die Linsenkörper eine vorgegebene Zeit in eine Pflege-, Reinigungs- und/oder Aufbewahrungslösung eingetaucht werden. Die Lösung enthält einen hornhautverträglichen, augenverträglichen und für den Körper verträglichen, abspülbaren, in die Matrix der Kontaktlinse eindringenden Farbstoff. Nach Ablauf der gewünschten Zeit werden die Linsenkörper aus dem Bad herausgenommen und mit einer klaren Lösung gespült.

Es ist auch denkbar, den Farbstoff mit einem geeigneten desinfizierenden oder neutralisierenden Mittel zu mischen, sodass der Benutzer sein Pflegemittel selbst herstellen kann, indem er es zum Beispiel mit Leitungswasser oder entionisiertem oder Leitungswasser mischt, wobei nach Möglichkeit durch Zugabe von Kochsalz eine isotonische Lösung angestrebt ist.

Nachstehend ein Beispiel für die erfindungsgemäße Lösung:

| | |
|---|---|
| Natriumchlorid (Isotonität) | 8 mg/ml |
| EDTA • 2NA (Chelatbildner) | 1 mg/ml |
| Borsäure (Puffer) | 5 mg/ml. |
| Natriumborat (Puffer) | 1 mg/ml |
| Entionisiertes Wasser | entsprechende Menge |
| E131, Patentblau V (blaue Farbe) | Menge abhängig vom Färbungsgrad |

Patentblau V oder E131 (CI 42051) ist ein künstlicher, in Wasser löslicher Farbstoff, der zu den Triphenylmethanfarbstoffen gehört. Der blauviolette Farbstoff wird vom menschlichen Organismus nicht aufgenommen. Er gilt als unbedenklich.

Nachstehend eine Reihe von Farbstoffen, die einzeln oder in Kombinationen zum Einfärben von Kontaktlinsen verwendet werden können:
- Methylene Blue CI 52015
- Victoria Blue CI 44045
- Methyl Violet CI 42535
- Rhodulin Blue CI 50220
- Chrysoidin CI 11270
- Malachite Green CI 42000
- FD&C Blue No. 1 CI 42090
- FD&C Green No. 3 CI 42053
- D&C Green No. 5 CI 61570
- Brilliant Green CI 42040
- Acid Blue No. 59 CI 50315
- Acid Blue No. 102 CI 50320
- Vat Green No. 1 CI 59825
- Vat Blue No. 6 CI 69825
- Vat Brown No. 1 CI 70800
- 1,4-BIS[2-(Methyphenyl) Amino] 9,10-Anthracenedione CAS 6737-68-4
- 4-[2,4-(Dimethyphenyl)azo]-2,4-dihydro-5-methyl-2-phenyl-3H-pyrazol-3-one CAS 6407-78-9
- 6-Ethoxy-2-(6-Ethoxy-3-Oxobenzo[b]Thien2(3H)-Ylidene)Benzo[b]Thiophen-3(2H)-one CAS 3263-31-8
- N,N-(9,10-Dihydro-9,10-dioxo-1,5-anthracenediyl) Bisbenzamide CAS 82-18-8.
- Acid Green No 50 CI 44090
- Acid Blue No 74 CI 73015
- Acid Yellow No 23 CI 19140
- Acid Red No 14 CI 14720
- Basic Violet No 3 CI 42555
- Basic Violet 14 CI 42510
- Basic Yellow No 3 CI 41005
- Basic Violet No 12 CI 50235

Die Einwirkzeit des Farbstoffs soll acht Stunden nicht übersteigen. Der Farbstoff ist naturgemäß molekular klein genug , damit er in den Linsenkörper auf die beschriebene Weise eindringen kann.

Ein weiterer Vorteil der Erfindung ist, dass auch Linsen eingefärbt werden können, die so als gefärbte Linsen nicht erhältlich sind, z.B. Speziallinsen wie Sonderanfertigungen, torische Linsen, Multifokallinsen etc. Diese werden industriell ausschließlich klar hergestellt.

## Patentansprüche

1. Pflege-, Reinigungs- und/oder Aufbewahrungslösung für Kontaktlinsen, **dadurch gekennzeichnet, dass** sie einen in der Pflege-, Reinigungs- und/oder Aufbewahrungslösung löslichen, für das Auge bzw. den Körper verträglichen, abspülbaren Farbstoff zum Einfärben der Kontaktlinsen enthält.

2. Farbstoff für eine Pflege-, Reinigungs- und/oder Aufbewahrungslösung von Kontaktlinsen, der in der Pflege-, Reinigungs- und/oder Aufbewahrungslösung lösbar, für das Auge und den Körper verträglich, abspülbar und geeignet ist, in einem Zeitraum von höchstens acht Stunden in die Matrix von Hydrogel-Linsen einzudringen und diese zu färben.

3. Farbstoff nach Anspruch 2, **dadurch gekennzeichnet, dass** er desinfizierend wirkt.

4. Farbstoff nach Anspruch 2, **dadurch gekennzeichnet, dass** er wundheilend für die Hornhaut wirkt.

5. Farbstoff nach Anspruch 2 oder 3, **dadurch gekennzeichnet, dass** er in gelöstem oder pulverförmigem Zustand ist.

6. Farbstoff nach Anspruch 2 oder 3, **dadurch gekennzeichnet, dass** er Tablettenform aufweist oder in einer Flüssigkeit löslichen Tablette enthalten ist.

7. Farbstoff nach einem der Ansprüche 2 bis 6, **dadurch gekennzeichnet, dass** er folgende Farben einzeln oder in Kombinationen enthält:
• Methylene Blue CI 52015
• Victoria Blue CI 44045
• Methyl Violet CI 42535
• Rhodulin Blue CI 50220
• Chrysoidin CI 11270
• Malachite Green CI 42000
• FD&C Blue No. 1 CI 42090
• FD&C Green No. 3 CI 42053
• D&C Green No. 5 CI 61570
• Brilliant Green CI 42040
• Acid Blue No. 59 CI 50315
• Acid Blue No. 102 CI 50320
• Vat Green No. 1 CI 59825
• Vat Blue No. 6 CI 69825
• Vat Brown No. 1 CI 70800
• 1,4-BIS[2-(Methyphenyl) Amino] 9,10-Anthracenedione CAS 6737-68-4
• 4-[2,4-(Dimethyphenyl)azo]-2,4-dihydro-5-methyl-2-phenyl-3H-pyrazol-3-one CAS 6407-78-9
• 6-Ethoxy-2-(6-Ethoxy-3-Oxobenzo[b]Thien2(3H)-Ylidene) Benzo[b] Thiophen-3(2H)-one CAS 3263-31-8
• N,N'-(9,10-Dihydro-9,10-dioxo-1,5-anthracenediyl) Bisbenzamide CAS 82-18-8.
• Acid Green No 50 CI 44090
• Acid Blue No 74 CI 73015
• Acid Yellow No 23 CI 19140
• Acid Red No 14 CI 14720
• Basic Violet No 3 CI 42555
• Basic Violet 14 CI 42510
• Basic Yellow No 3 CI 41005
• Basic Violet No 12 CI 50235

8. Verfahren zum Färben von Hydrogel-Kontaktlinsen, insbesondere mit einem Farbstoff nach einem der Ansprüche 2 bis 7, **gekennzeichnet durch** folgende Schritte:
- die Linsenkörper werden für eine vorgegebene Zeit in eine Pflege-, Reinigungs- und/oder Aufbewahrungslösung eingetaucht, die gelöst einen augenverträglichen bzw. körperverträglichen, abspülbaren, in die Matrix der Kontaktlinse eindringenden Farbstoff enthält und
- anschließend werden die Linsenkörper mit klarer Lösung gespült.
